# EUROPEAN PATENT SPECIFICATION

(11) **EP 0 680 322 B1**
(45) Date of publication and mention of the grant of the patent: **05.07.2000**
(21) Application number: 94904707.0
(22) Date of filing: 21.01.1994
(51) Int. Cl.: A61K 31/42, A61K 31/545, A61K 31/43

(54) **USE OF CLAVULANATE AND AN ANTIBACTERIAL COMPOUND FOR THE TREATMENT OF INFECTIONS**
VERWENDUNG VON CLAVULANSÄURE UND EINEM ANTIBIOTIKUM ZUR BEHANDLUNG VON INFEKTIONEN
UTILISATION DE CLAVULANATE ET UN ANTIBIOTIQUE POUR LE TRAITEMENT D'INFECTIONS

(30) Priority: 22.01.1993 GB 9301250; 13.02.1993 GB 9302908
(43) Date of publication of application: 08.11.1995
(60) Divisional application: 00201268.0
(73) Proprietor: SMITHKLINE BEECHAM PLC, Brentford, Middlesex TW8 9EP (GB)
(72) Inventor: SMITH, Gillian Marjory, Science Park South,Harlow,ESSEX CM19 5AW (GB); THORBURN, Christine Elaine, cience Park South, Harlow, ESSEXCM195AW (GB); ABBOTT, Karen Hazel, Science PArk South,Harlow,EssexCM195AW (GB); BROWN, Tim Neilc/0 Smithkline Beecham Pharmaceutic, CM195AW (GB)
(74) Representative: Connell, Anthony Christopher
(86) International application number: GB9400124
(87) International publication number: WO9416696

(56) References cited:
- FR-A- 2 267 778
- PROCEEDINGS FIRST SYMPOSIUM AUGMENTIN: EXCERPTA MEDIA INT. CONG. SER. 1980 pages 80 - 83 GREENWOOD, D. 'SHORT COMMUNICATION: A TYPE OF INTERACTION BETWEEN CLAVULANIC ACID AND OTHER BETA-LACTAM ANTIBIOTICS DISTINCT FRON BETA-LACTAMASE INHIBITION EFFECT' cited in the application
- JOURNAL OF CHEMOTHERAPY vol. 5, no. 3 , June 1993 pages 147 - 150 SEGATORE, B. ET AL 'IN VITRO ACTIVITY OF CEFODIZIME (HR-221) IN COMBINATION WITH BETA-LACTAMASE INHIBITORS'
- EUROPEAN JOURNAL OF CLINICAL MICROBIOLOGY AND INFECTIOUS DISEASES vol. 10, no. 2 , February 1991 pages 77 - 84 SUMITA, Y. ET AL 'IN VITRO SYNERGISTIC ACTIVITY BETWEEN MEROPENEM AND OTHER BETA-LACTAMS AGAINST METHICILLIN-RESISTANT STAPHYLOCOCCUS AUREUS'

## Description

This invention relates to pharmaceutical formulations, in particular to formulations for the treatment of bacterial infections.

Clavulanic acid is a known compound having efficacy in inhibiting bacterial β-lactamase enzymes, which degrade β-lactam antibiotics such as penicillins and confer resistance to such antibiotics. Clavulanic acid in the form of its derivatives (hereinafter termed "clavulanate"), particularly its salts, are consequently used in formulations in combination with β-lactam antibiotics, for example as described in GB 2005538, to suppress the activity of β-lactamase enzymes which mediate bacterial resistance to β-lactam antibiotics.

The possibility of clavulanic acid inhibiting bacterial resistance mechanisms other than β-lactamase-mediated has been suggested, e.g. D. Greenwood, Proc. Ist. Symp. Augmentin: Excerpta Med. Int. Cong. Ser. (1980) 80-83. This publication only describes *in vitro* work on *E. coli*, and the β-lactam antibiotics cephalexin, cephadrine, and mecillinam. This publication concludes that the observed effect is unlikely to be of any general therapeutic significance.

The inventors have discovered an unexpected further activity of clavulanic acid in enhancing the effectiveness of antibacterial compounds against microorganisms which have an antibiotic resistance mechanism which is different to that mediated by β-lactamase enzymes. This effect is observed *in vivo*, and may be of therapeutic significance in the therapy of infections by β-lactamase negative penicillin resistant pathogens such as *S. pneumoniae and H. influenzae.*

The present invention provides a method of use of clavulanate and an antibacterial compound in the manufacture of a medicament formulation for the treatment of infection of humans or animals by microorganisms having a resistance mechanism other than β-lactamase mediated resistance, with the proviso that the antibacterial compound is not ampicillin or cephaloridine and the infection is not caused by *Staphylococcus Aureus* microorganisms.

The above use is particularly suitable in respect of penicillin-resistant microorganisms, e.g. Which are believed to have a penicillin-binding-protein (hereinafter termed "PBP") mediated resistance mechanism, although the exact mechanism of the resistance mechanism inhibited by clavulanate in the present invention is not known, and the invention is not limited to any specific mechanism.

This type of microorganism includes penicillin-resistant organisms such as *Streptococcus spp*., e.g. *S. pneumoniae, Haemophilus spp.*, *e.g. H. influenzae*, *Staphylococcus spp*., except *staphylococcus aureus, Enterococcus spp*., and *Neisseria spp*., e.g. *N. gonococcus* and *N. meningitidis.* Of these, *S. pneumoniae* and *H. influenzae* are major pathogens.

Typically the clavulanate may be present as a salt, preferably as its potassium salt, ie potassium clavulanate. The clavulanate may in some cases be antibacterially effective by itself against the organisms, but preferably the clavulanate is used in the above uses, formulations and methods of this invention in combination with an antibacterial agent, for example an antibiotic, suitably a β-lactam antibiotic such as a penicillin or cephalosporin. Preferably the antibacterial agent is a β-lactam antibiotic.

Suitable β-lactam antibiotics include the penicillins: amoxycillin, apalcillin, aspoxicillin, azidocillin, azlocillin, aztreonam, benzylpenicillin, bacampicillin, carbenicillin, cloxacillin, cyclacillin, dicloxicillin, epicillin, flucloxacillin, lenampicillin, mecillinam, methicillin, mezlocillin, phenoxymethylpenicillin, piperacillin, pivampicillin, propicillin, sulbenicillin, talampicillin, and ticarcillin; and the cephalosporins: cefaclor, cefadroxil, cefatrizine, cefclidine, cefamandole, cefazolin, cefbuperazone, cefcanel daloxate, cefdinir, cefepime, cefetamet pivoxil, cefixime, cefminox, cefminoxime,. cefmetazole, cefonicid, cefoperazone, cefotaxime, cefotetan, cefotiam, cefotiam hexetil, cefoxitin, cefpimizole, cefpiramide, cefpirome, cefpodoxime proxetil ,cefprozil, ceftazidime, ceftibuten, ceftizoxime, ceftriaxone, cefuroxime axetil, cefuroxime, cephacetrile, cephalexin, cephalothin, cephamanadole nafate, cephapirin, cephoperazone, cefsulodin, cefuzonam, cephradine, loracarbef, DQ 2556, ME1207, S-1006, SCE-2787 and moxalactam.

Particularly suitable β-lactam antibiotics include the penicillins: amoxycillin, apalcillin, aspoxicillin, azidocillin, azlocillin, aztreonam, benzylpenicillin, bacampicillin, carbenicillin, cloxacillin, cyclacillin, dicloxicillin, epicillin, flucloxacillin, lenampicillin, mecillinam, methicillin, mezlocillin, phenoxymethylpenicillin, piperacillin, pivampicillin, propicillin, sulbenicillin, talampicillin, and ticarcillin; and the cephalosporins: cefaclor, cefadroxil, cefatrizine, cefclidine, cefamandole, cefazolin, cefbuperazone, cefcanel daloxate, cefdinir, cefepime, cefetamet pivoxil, cefminox, cefminoxime, cefmetazole, cefonicid, cefoperazone, cefotaxime, cefotetan, cefotiam, cefotiam hexetil, cefoxitin, cefpimizole, cefpiramide, cefpirome, cefprozil, ceftazidime, ceftibuten, ceftizoxime, ceftriaxone, cephacetrile, cephalexin, cephalothin, cephamanadole nafate, cephapirin, cephoperazone, cefsulodin, cefuzonam, cephradine, DQ 2556, ME1207, S-1006, SCE-2787 and moxalactam.

The β-lactam antibiotics referred to herein may be in the form of the free acids or pharmaceutically acceptable salts or in-vivo hydrolysable esters.

Preferred antibacterial agents include amoxycillin, suitably in the form of amoxycillin trihydrate for oral use, and in the form of sodium amoxycillin for parenteral use, and the cephalosporins cefaclor or cefprozil.

The clavulanate and any other antibacterial agent such as the penicillin or cephalosporin antibiotics, as used in this invention, whether in the form of the free acids, salts, esters or derivatives thereof are preferably each in a substantially pure form, e.g. at least 60% pure, more suitably at least 75% pure, preferably at least 85% especially at least 98% pure on a weight basis.

In the methods of this invention, clavulanate and an antibacterial agent such as the penicillin or cephalosporin antibiotics, e.g amoxycillin, cefaclor or cefprozil, may be administered together, simultaneously, successively or in any order, but typically may be administered together as a co-formulation.

The formulation may be formulated for administration by any route, such as oral, topical or parenteral. The route of choice may for example be determined by the route of choice for the antibacterial agent used in combination with the clavulanate. The formulation may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present at from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. Such tablets may also include an effervescent couple of generally known type, e.g a solid carboxylic acid and an alkali metal carbonate or bicarbonate. Such tablets may also include a chewable base such as mannitol, sorbitol or lactose, optionally together with an effervescent couple, for example as described in EP 0389177. Such tablets and solid dosage forms may be made by any of the generally known methods for such dosage forms, and may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing clavulanate and any antibacterial agent and a sterile vehicle, water being preferred. These active compounds, depending on the vehicle and concentration used, can be either suspended or dissolved in the vehicle. In preparing solutions the active compounds can be dissolved in water for injection and filter sterilised before filling into a suitable vial or ampoule and sealing.

Advantageously, agents such as a local anaesthetic, preservative and buffering agents can be dissolved in the vehicle. To enhance the stability, the formulation can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the ingredients of the suspension are suspended in the vehicle instead of being dissolved and sterilization cannot be accomplished by filtration. The formulation can be sterilised by exposure of its dry constituents to ethylene oxide before suspending in the sterile vehicle. Advantageously, a surfactant or wetting agent is included in the formulation to facilitate uniform distribution of the active compounds.

Since salts of clavulanic acid are extremely hygroscopic the solid and non-aquous liquid formulations of this invention must be prepared in dry conditions, typically at a relative humidity of 30% or less. All constituents of formulations of this invention should be predried. Aqueous solution and suspension formulations of this invention can only be provided in the form of dry solids for make up into aqueous solution or suspension shortly prior to use, for example 5 days in the case of oral suspensions. It may also be necessary to maintain such suspensions at low temperatures, e.g >5°C.

In view of the extreme moisture sensitivity of clavulanate, aqueous suspensions or solutions insofar as they contain clavulanate must be provided as dry solids for reconstitution with water shortly before administration.

A formulation according to the invention may be in unit dosage form, for example unit dosage form for oral or parenteral administration, which latter will primarily include administration by injection or infusion, especially intramuscular and intravenous administration.

The above-mentioned formulations may contain 0.1 - 90% by weight, preferably from 10 - 60% by weight of the active materials, depending on the method of administration.

The clavulanate may suitably be administered to the patient at a daily dosage of from 0.3 to 15 mg/kg, preferably from 0.7 to 10 mg/kg, for example from 0.7 to 7 mg/kg, of body weight. For an adult human (of approximately 70 kg body weight), from 25 to 1000 mg, preferably from 50 to 500 mg, of clavulanate expressed as its free acid equivalent may be administered daily, suitably in from 1 to 6, preferably from 2 to 4, separate doses. Higher or lower dosages may, however, be used in accordance with clinical practice.

When the formulations according to the invention are presented in unit dosage form, each unit dose may suitably comprise from 12.5 to 1000 mg, preferably from 12.5 to 250 mg, of clavulanate. Each unit dose may, for example, be 12.5, 25, 50, 75, 100, 125, 150, 200, or 250 mg of clavulanate.

The ratio of the amount of the clavulanate used according to the invention to the amount of any antibacterial agent present may vary within a wide range. In a pharmaceutical or medicament formulation of this invention the said ratio may, for example, be from 1:1 to 1:30; more particularly, it may, for example, be from 1:1 to 1:12, for example 1:2, 1:3, 1:4,1:5, 1:6, 1:7, 1:8, or 1:9 by weight, suitably within a variance of ± 10%.

Suitable unit dosages and maximum daily dosages of any antibacterial agent used in combination with clavulanate in this invention may for example be determined according to the unit dosages and maximum daily dosages of the agent used conventionally. For example amoxycillin is generally provided in unit dosages of 125 to 1000 mg, administered from 2 to 4 times daily to a typical daily dosage of 125 to 3000 mg per day. For example cefaclor is generally provided in unit dosages of 250 and 500 mg, and may be dosed up to a maximum daily dosage of 4000 mg per day.

A preferred combination of this invention is clavulanate with amoxycillin, in a ratio clavulanate : amoxycillin in the range 1:1 to 1:12, for example together in a formulation. An example of a suitable formulation according to the invention for oral administration is one comprising from 125 to 3000 mg, preferably from 500 to 1000 mg, of amoxycillin trihydrate, in admixture or conjunction with from 12.5 to 250 mg, preferably from 25 to 125 mg, of potassium clavulanate per unit dose.

A further example of a suitable formulation according to this invention for parenteral administration is one comprising from 125 to 3000 mg of sodium amoxycillin, in admixture or conjunction with from 12.5 to 250 mg, preferably from 25 to 125 mg, of potassium clavulanate.

An example of a unit dosage form of a formulation of this invention comprises 12.5 to 1000 mg of potassium clavulanate and 62.5 to 500 mg of cefaclor.

The following examples illustrate the antibacterial activity of amoxycillin, cefaclor and cefprozil, and clavulanate in combination and compare it with the activity of amoxycillin alone against a penicillin-resistant strain of *Streptococcus pneumoniae.*

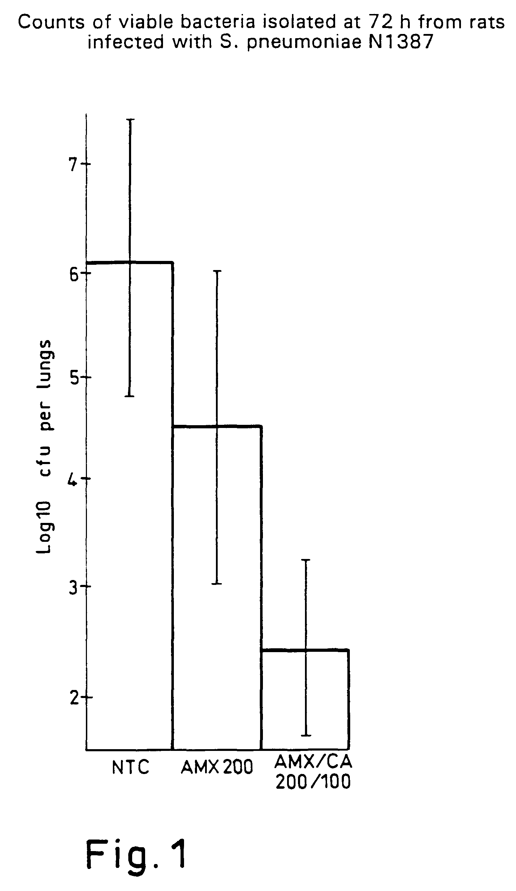

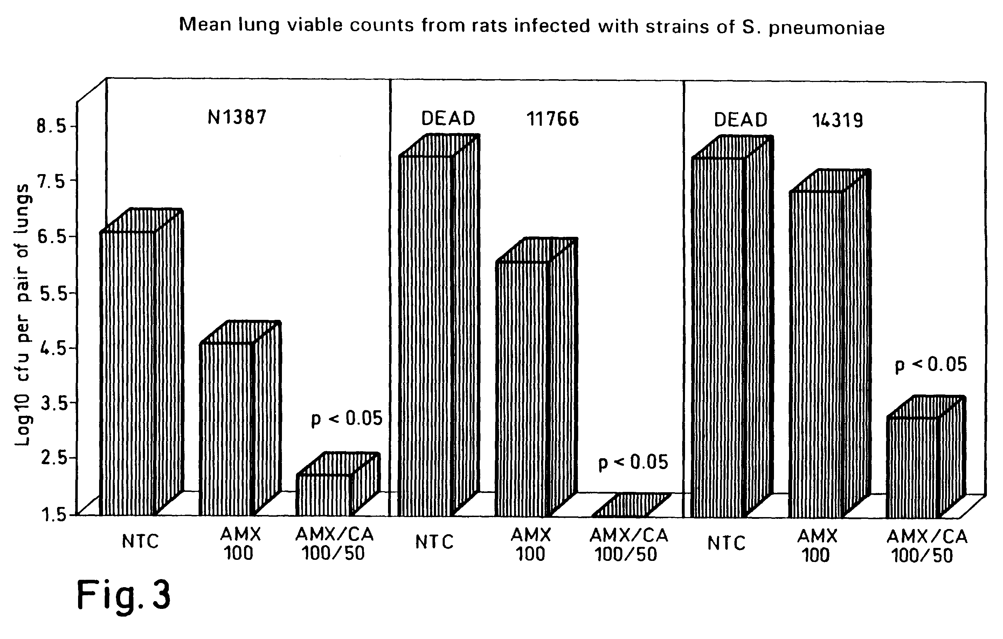

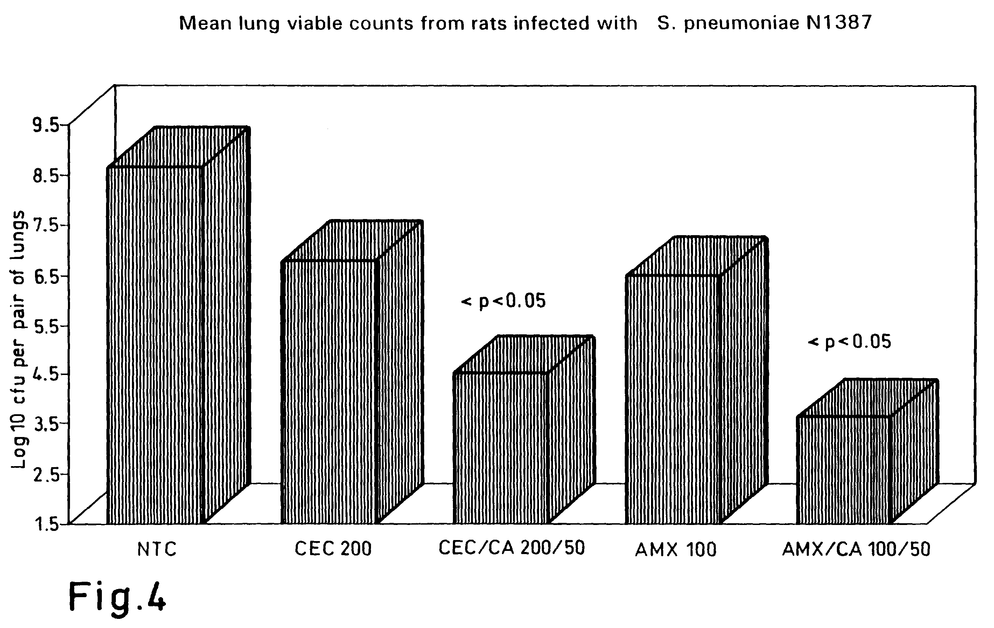

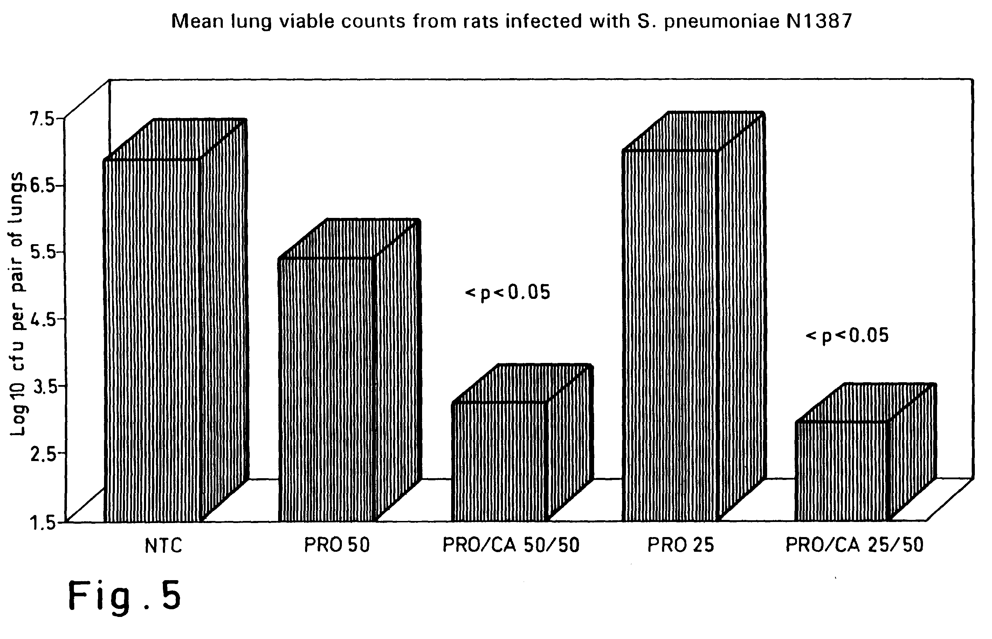

Figs. 1 to 5 show graphically the level of *S. pneumoniae* growth in vivo following administration of amoxycillin : clavulanate, cefaclor : clavulanate and cefprozil : clavulanate compared with comparisons and controls. In these examples the abbreviations NTC = non treated control, AMX = amoxycillin, CA = potassium clavulanate, CEC = cefaclor and PRO = cefprozil are used, and doses of clavulanate and the antibiotics in mg/kg are shown in the graphs against Log₁₀ cfu per pair of lungs.

### Example 1

### Summary

A model of a penicillin-resistant *Streptococcus pneumoniae* respiratory infection in immunocompromised rats was developed for comparative efficacy studies with antibacterials. Rats were rendered neutropenic with cyclophosphamide, and infected by intrabronchial instillation of a penicillin-resistant strain of *S.pneumoniae.*

The infection persisted in the rats' lungs for at least four days at a mean count of 7.0 log 10 cfu/lungs, although the mortality rate was low. Oral therapy commenced 24 h post infection and continued q12h for three days. Assessment of therapy was by counts of bacteria recovered from lung samples at intervals during the studies.

Amoxycillin (200 mg/kg) showed little activity against this strain, but amoxycillin/clavulanate (200/100 mg/kg) was effective in reducing numbers of *S.pneumoniae* from the lungs within 48 h of therapy, and with a further significant reduction to 2-3 log 10 cfu/lungs at 72 h and 96 h.

### Materials and Methods

Animals: Weanling male specific pathogen free ("SPF") rats (60-80 g, CD strain) were supplied by Charles River UK Ltd.

Induction of leukopenia: Rats were dosed intraperitoneally with 0.5 ml cyclophosphamide (Endoxana, Boehringer Ingelheim Ltd., Bracknell). at 50 mg/kg three days before, and on the day of infection.

Organism: *S.pneumoniae* N1387 was used in both studies.

Inoculum: A stock inoculum of *S.pneumoniae* N1387 (stored at-70°C) was grown on blood agar at 37°C and the growth from six plates was suspended in 3 ml Todd Hewitt broth (TH). This was further diluted 1:5 in molten nutrient agar maintained at 40°C.

Anaesthesia: Rats were anaesthetised by separate intramuscular injections of 50µl of fentanyl fluanisone at 0.1 ml/kg (Hypnorm, Janssen Pharmaceuticals Ltd., Grove), diazepam at 0.5 mg/kg (Valium, Roche products Ltd,. Welwyn Garden City). The drugs were prepared in sterile distilled water.

Infection: Anaesthetised rats were infected by intrabronchial instillation of a 50µl inoculum containing 6-7 log 10 cfu *S. pneumoniae* by means of non-surgical intratracheal intubation.

Compounds: Amoxycillin trihydrate and potassium clavulanate (SmithKline Beecham Pharmaceuticals, Worthing) were dissolved in pH 8.0 phosphate buffer and sterile distilled water respectively.

Dosage: Groups of 5 rats received 0.5 ml of each agent by oral gavage. Therapy commenced 24 h post infection, and continued b.i.d. (q12h) for three days. Rats received amoxycillin alone at 200 mg/kg or amoxycillin/clavulanic acid at 200/100 mg/kg to give AUCs in plasma equivalent to those produced in man following a 500 mg dose of amoxycillin or a 625 mg dose of Augmentin (500 mg amoxycillin plus 125 mg potassium clavulanate) respectively.

### Results.

An initial study was performed to look at the effects of amoxycillin and amoxycillin/clavulanate following two days of twice-daily therapy (q12h), and lung bacterial counts were taken at 72 h only (Figure 1). In this study, following infection with 7.0 log 10 cfu *S. pneumoniae* per rat, amoxycillin/clavulanate was effective in significantly reducing bacterial numbers by 72 h in comparison with the non-treated controls (p=<0.05). Following therapy with amoxycillin alone, some reduction in bacterial numbers was noted, although counts were not significantly different from those in the non-treated control group (p=>0.5). In this study, amoxycillin/clavulanate was significantly different from amoxycillin (p less than 0.05).

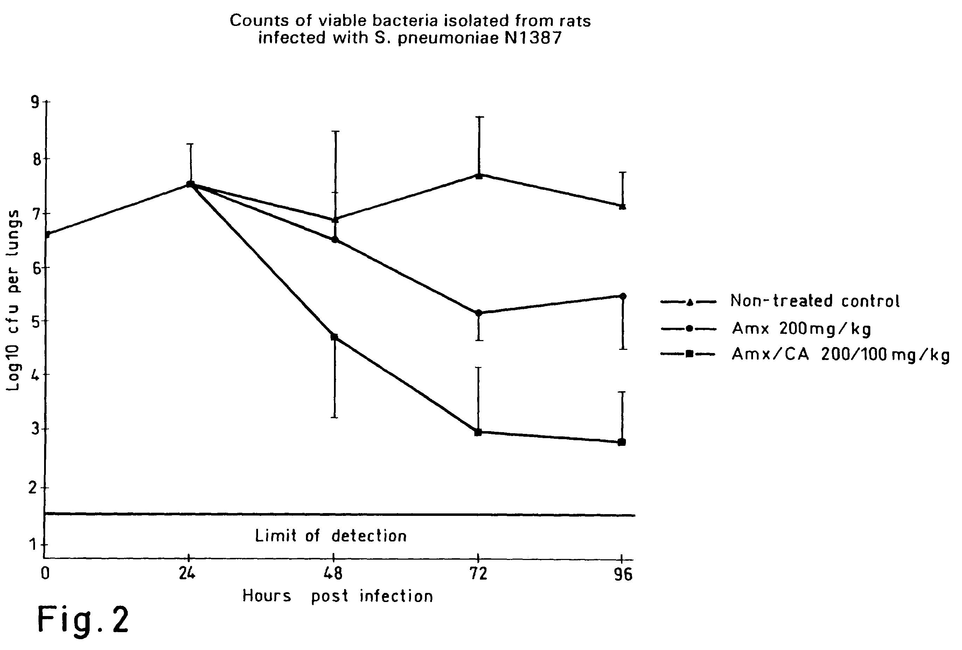

A further study was carried out in order to confirm these findings, and to determine the course of infection over a four day period. The results are shown in Figure 2. Following infection with 6.6 log 10 cfu per rat, *S. pneumoniae* grew well in the rats' lungs, reaching 7.6±1.0 log 10 cfu/lungs in the non-treated group by 72 h, with 7.0±0.6 log 10 cfu/lungs persisting at 96h post infection. Amoxycillin again had some effect on the infection, and significantly reduced numbers of the organism to 5.0±0.5 by 72h, although at 96 h the counts were not significantly different from those in the non-treated control group. Amoxycillin/clavulanate, however, caused a more rapid reduction in numbers, with 4.6±1.5 log 10 cfu/lungs detectable at 48 h, and with a further reduction by 72 h-96 h with only 2.8±1.2 and 2.6±0.9 log 10 cfu/lungs detectable respectively. In this study, amoxycillin/clavulanate was again significantly more effective than amoxycillin alone (p=<0.05).

### Discussion

These results demonstrate that, in this rat respiratory model, twice daily oral therapy with amoxycillin/clavulanate at 200/100 mg/kg (625 mg equivalent in man) was significantly more effective than amoxycillin at 200 mg/kg (500 mg equivalent in man) in reducing numbers of a penicillin- and macrolide- resistant strain of *S. pneumoniae* from the lungs of infected animals.

### Example 2

A further experiment was carried out along similar lines to Example 1, again using specific pathogen-free rats. As well as therapy with potassium clavulanate : amoxycillin, therapy with potassium clavulanate : cefaclor and potassium clavulanate : cefprozil was also investigated.

As a control, amoxycillin alone and in the presence of clavulanate was tested, and no loss of active amoxycillin was detected in the site of infection, i.e. the lungs of rats treated with amoxycillin alone or with clavulanate, confirming the absence of β-lactamase-producing organisms. Synergy between amoxycillin and clavulanate was also shown using an intraperitoneal infection, which is normally a sterile site, negating the possibility of other bacteria interfering with the test.

The results of these experiments are shown graphically in Figs. 3, 4 and 5.

Fig. 3. shows that amoxycillin : clavulanate was significantly more effective than amoxycillin alone against three penicillin resistant strains of *S. pneumonia*

Fig. 4 shows that cefaclor : potassium clavulanate was significantly more effective than cefaclor alone against penicillin resistant *S. pneumoniae* N1387, showing effectiveness at cefaclor : clavulanate 200 : 50 mg/kg.

Fig. 5 shows that cefprozil : potassium clavulanate was significantly more effective than cefprozil alone against penicillin resistant *S. pneumoniae* N1387, showing effectiveness at cefprozil : clavulanate 50 : 50 mg/kg and 25 : 50 mg/kg.

### Example 3.

### Method.

Determinations of the minimum inhibitory concentration (MIC) were performed in agar using serial dilutions of the test compounds alone or in the presence of constant concentrations of clavulanic acid (2 or 4 µg/ml). The agar used was Mueller Hinton (BBL) supplemented with 5% lysed horse blood in the case of *H. influenzae* and *M. catarrhalis*, except for *B. fragilis* where Wilkins-Chalgren agar (Oxoid) was used. A multipoint inoculator was used to drop 1µl of undiluted culture of *S. aureus*, 10-fold dilutions of *B. fragilis*, *H. influenzae* and *M. catarrhalis* and 100-fold dilutions of *E. coli* and *K. pneumoniae* onto the surface of the agar, to give an inoculum of approximately 10⁴ to 10⁵ cfu/spot.

### Results

The activities of cefaclor ("Distaclor" - Trade Mark (Dista) Lot No 64473ae (96% pfa)) alone and in the presence of clavulanic acid are illustrated in Table 1. Against β-lactamase producing strains of *S. aureus*, 2 and 4 µg/ml clavulanic acid markedly improved the activity of cefaclor, reducing the geometric mean (Gmean) MIC's from 12.44 to 1.13 and 0.73 µg/ml respectively. The activity of cefaclor against plasmid mediated β-lactamase producing strains of *E. coli* and *K. pneumoniae* was also improved by clavulanic acid, the Gmean MIC values being reduced considerably (Table 1). Against β-lactamase producing strains of *M. catarrhalis*, *H. influenzae* and the strains of *B. fragilis* tested, clavulanic acid at a concentration of 2 or 4 µg/ml improved the activity of cefaclor.

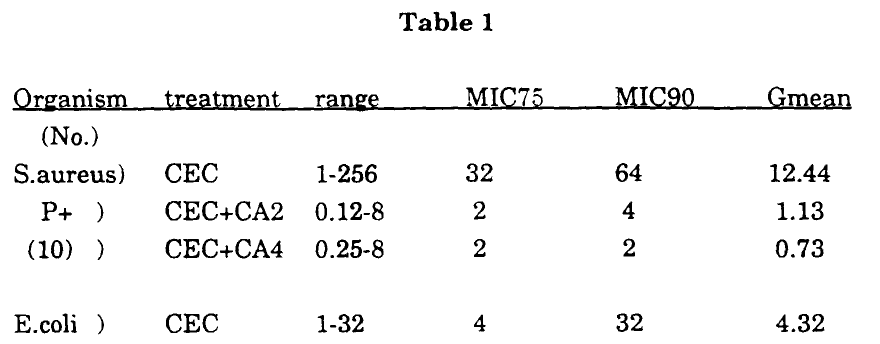

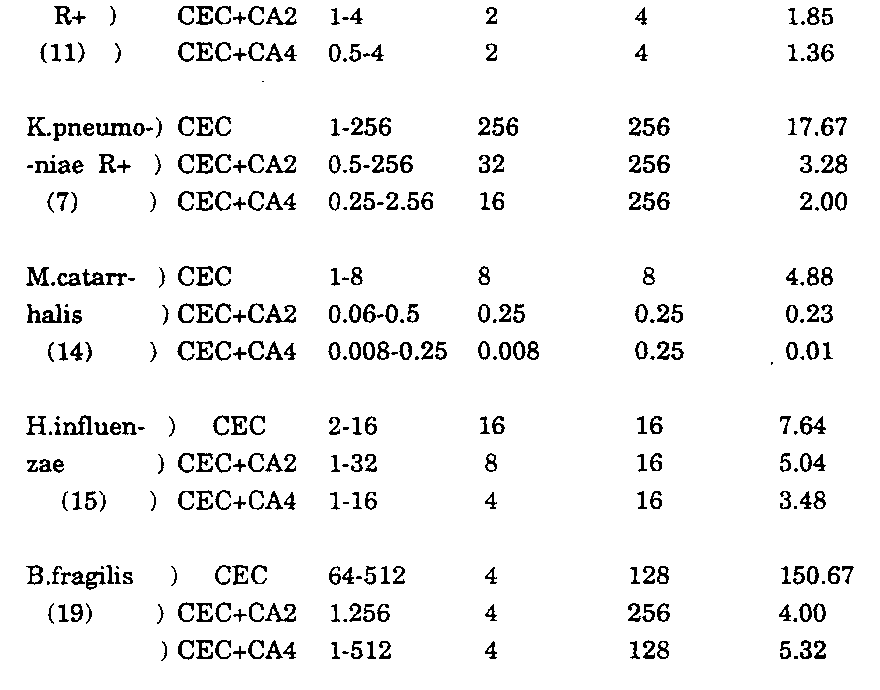

## Claims

1. Use of clavulanate and an antibacterial compound in the manufacture of a medicament formulation for the treatment of infection of humans or animals by microorganisms having a resistance mechanism other than beta-lactamase mediated resistance, with the proviso that the antibacterial compound is not ampicillin or cephaloridine and the infection is not caused by *Staphylococcus Aureus* microorganisms.

2. Use according to claim 1 characterised in that the mechanism of resistance is penicillin-binding-protein (hereinafter termed "PBP") mediated resistance mechanism.

3. Use according to any one of claims 1 or 2 characterised in that the microorganisms we penicillin-resistant organisms.

4. Use according to any one of claims 1 to 3 characterised in that the microorganisms we selected from *Streptococcus spp*., *Haemophilus spp*., *Staphylococcus spp*., *Enterococcus spp*., and *Neisseria spp*. or *N. meningitidis.*

5. Use according to any one of the preceding claims characterised in that the clavulanate is potassium clavulanate.

6. Use according to any one of the preceding claims characterised in that the antibacterial agent is a beta-lactam antibiotic selected from the penicillins: apalcillin, aspoxicillin, azidocillin, azlocillin, aztreonam, benzylpenicillin, bacampicillin, carbenicillin, cloxacillin, cyclacillin, dicloxicillin, epicillin, flucloxacillin, lenampicillin, mecillinam, methicillin, mezlocillin, phenoxymethylpenicillin, piperacillin, pivampicillin, propicillin, sulbenicillin, talampicillin, and ticarcillin; and the cephalosporins: cefaclor, cefadroxil, cefatrizine, cefclidine, cefamandole, cefazolin, cefbuperazone, cefcanel daloxate, cefdinir, cefepime, cefetamet pivoxil, cefixime, cefminox, cefminoxime, cefmatazole, cefonicid, cefoperazone, cefotaxime, cefotetan, cefotiam, cefotiam hexetil, cefoxitin, cefpimizole, cefpiramide, cefpirome, cefpodoxime proxetil, cefprozil, ceftazidime, ceftibuten, ceftizoxime, ceftriaxone, cefuroxime axetil, cefuroxime, cephacetrile, cephalexin, cephalothin, cephamanadole nafate, cephapirin, cephoperazone, cefsulodin, cefuzonam, cephradine, loracarbef, DQ2556, ME1207, S-1006, SCE-2787 and moxalactam.

7. Use according to any of claims 1 to 5 characterised in that the antibacterial agent is amoxycillin.

## Patentansprüche

1. Verwendung von Clavulanat und einer antibakteriellen Verbindung für die Herstellung einer Medikamentenformulierung für die Behandlung einer Infektion von Menschen oder Tieren durch Mikroorganismen mit einem Resistenzmechanismus, der keine β-Lactamase-vermittelte Resistenz ist, mit der Maßgabe, daß die antibakterielle Verbindung nicht Ampicillin oder Cephaloridin ist und die Infektion nicht durch *Staphylococcus aureus*-Mikroorganismen verursacht wird.

2. Verwendung nach Anspruch 1, dadurch gekennzeichnet, daß der Resistenzmechanismus ein durch Penicillin-Bindungsprotein (nachstehend mit "PBP" bezeichnet) vermittelter Resistenzmechanismus ist.

3. Verwendung nach einem der Ansprüche 1 oder 2, dadurch gekennzeichnet, daß die Mikroorganismen Penicillin-resistente Organismen sind.

4. Verwendung nach einem der Ansprüche 1 bis 3, dadurch gekennzeichnet, daß die Mikroorganismen ausgewählt sind aus *Streptococcus spp*., *Haemophilus spp*., *Staphylococcus spp*., *Enterococcus spp. und Neisseria spp. oder N. meningitidis.*

5. Verwendung nach einem der vorangehenden Ansprüche, dadurch gekennzeichnet, daß das Clavulanat Kaliumclavulanat ist.

6. Verwendung nach einem der vorangehenden Ansprüchen, dadurch gekennzeichnet, daß das antibakterielle Mittel ein β-Lactam-Antibiotikum ist, ausgewählt aus den Penicillinen: Apalcillin, Aspoxicillin, Azidocillin, Azlocillin, Aztreonam, Benzylpenicillin, Bacampicillin, Carbenicillin, Cloxacillin, Cyclacillin, Dicloxicillin, Epicillin, Flucloxacillin, Lenampicillin, Mecillinam, Methicillin, Mezlocillin, Phenoxymethylpenicillin, Piperacillin, Pivampicillin, Propicillin, Sulbenicillin, Talampicillin und Ticarcillin; und den Cephalosporinen: Cefaclor, Cefadroxil, Cefatrizin, Cefclidin, Cefamandol, Cefazolin, Cefbuperazon, Cefcanel-Daloxat, Cefdinir, Cefepim, Cefetamet-Pivoxil, Cefixim, Cefminox, Cefminoxim, Cefmatazol, Cefonicid, Cefoperazon, Cefotaxim, Cefotetan, Cefotiam, Cefotiam-Hexetil, Cefoxitin, Cefpimizol, Cefpiramid, Cefpirom, Cefpodoxim-Proxetil, Cefprozil, Ceftazidim, Ceftibuten, Ceftizoxim, Ceftriaxon, Cefuroxim-Axetil, Cefuroxim, Cephacetril, Cephalexin, Cephalothin, Cephamanadol-Nafat, Cephapirin, Cephoperazon, Cefsulodin, Cefuzonam, Cephradin, Loracarbef, DQ2556, ME1207, S-1006, SCE-2787 und Moxalactam.

7. Verwendung nach einem der Ansprüche 1 bis 5, dadurch gekennzeichnet, daß der antibakterielle Wirkstoff Amoxycillin ist.

## Revendications

1. Utilisation de clavulanate et d'un composé antibactérien dans la production d'une formulation de médicament pour le traitement d'une infection chez l'homme ou des animaux par des micro-organismes présentant un mécanisme de résistance autre qu'une résistance à médiation par la bêta-lacamase, sous réserve que le composé antibactérien ne consiste en ampicilline ou céphaloridine et que l'infection ne soit pas provoquée par des micro-organismes *Staphylococcus aureus.*

2. Utilisation suivant la revendication 1, caractérisée en ce que le mécanisme de résistance est un mécanisme de résistance à médiation par la protéine de liaison à la pénicilline (appelée ci-après "PBP").

3. Utilisation suivant l'une quelconque des revendications 1 et 2, caractérisée en ce que les micro-organismes sont des micro-organismes résistant à la pénicilline.

4. Utilisation suivant l'une quelconque des revendications 1 à 3, caractérisée en ce que les micro-organismes sont choisis entre *Streptococcus spp*., *Haemophilus spp*., *Staphylococcus spp*., *Enterococcus spp*., et *Neisseria spp*. ou *N. meningitidis*.

5. Utilisation suivant l'une quelconque des revendications précédentes, caractérisée en ce que le clavulanate est le clavulanate de potassium.

6. Utilisation suivant l'une quelconque des revendications précédentes, caractérisée en ce que l'agent antibactérien est un antibiotique du type bêta-lactame choisi parmi les pénicillines : apalcilline, aspoxicilline, azidocilline, azlocilline, aztréonam, benzylpénicilline, bacampicilline, carbénicilline, cloxacilline, cyclacilline, dicloxicilline, épicilline, fluc.oxacilline, lénampicilline, mécillinam, méthicilline, mezlocilline, phénoxyméthylpénicilline, pipéracilline, pivampicilline, propicilline, sulbéncicilline, talampicilline, et ticarcilline, et les céphalosporines : céfaclor, céfadroxil, céfatrizine, cefclidine, céfamandole, céfazoline, cefbupérazone, cefcanel, daloxate, cefdinir, céfépime, cefetamet pivoxil, céfixime, cefminox, cefminoxime, cefmatazole, céfonicide, cefoperazone, céfotaxime, céfotétan, céfotiam, céfotiam, hexétil, céfoxitine, cefpimizole, cefpiramide, cefpirome, cefpodoxime proxétil, cefprozil, ceftazidime, ceftibutène, ceftizoxime, ceftriaxone, cefuroxime axétil, céfuroxime, céphacétrile, céphalexine, céphalothine, céphamandole nafate, céphapirine, céphopérazone, cefsulodine, céfuzonam, céphradine, loracarbef, DQ2556, ME1207, S-1006, SCE-2787 et moxalactame.

7. Utilisation suivant l'une quelconque des revendications 1 à 5, caractérisée en ce que l'agent antibactérien consiste en amoxycilline.
